# EUROPEAN PATENT SPECIFICATION

(11) **EP 4 488 672 B1**
(45) Date of publication and mention of the grant of the patent: **03.09.2025**
(21) Application number: 23306132.4
(22) Date of filing: 05.07.2023
(51) Int. Cl.: G01N 27/414, G01N 33/543, G01N 33/70

(54) **ORGANIC ELECTROCHEMICAL SENSOR WITH MUTUALIZED ELECTRODES AND METHOD FOR MEASURING**
ORGANISCHER ELEKTROCHEMISCHER SENSOR MIT VERGEMEINSCHAFTETEN ELEKTRODEN UND MESSVERFAHREN
CAPTEUR ÉLECTROCHIMIQUE ORGANIQUE À ÉLECTRODES MUTUALISÉES ET PROCÉDÉ DE MESURE

(43) Date of publication of application: 08.01.2025
(73) Proprietor: Omini, 59120 Loos (FR)
(72) Inventor: ROHTLAID, Katlin, 59120 Loos (FR); PREIRA, Pascal, 59120 Loos (FR); ARRUABARRENA, Jean-Francois, 59120 Loos (FR); MARTY, Alain, 59120 Loos (FR); KANAAN, Joanne, 59120 Loos (FR); SHIRINSKAYA, Anna, 59120 Loos (FR)
(74) Representative: Icosa

(56) References cited:
- EP-A1- 3 045 902
- US-B1- 6 241 863
- DEMURU SILVIA ET AL: "Real-Time Multi-Ion Detection in the Sweat Concentration Range Enabled by Flexible, Printed, and Microfluidics-Integrated Organic Transistor Arrays", ADVANCED MATERIALS TECHNOLOGIES, vol. 5, no. 10, 16 August 2020 (2020-08-16), DE, XP093104503, ISSN: 2365-709X, Retrieved from the Internet <URL:https://onlinelibrary.wiley.com/doi/full-xml/10.1002/admt.202000328> [retrieved on 20231122], DOI: 10.1002/admt.202000328
- LIN BAOJUN ET AL: "Flexible organic integrated electronics for self-powered multiplexed ocular monitoring", NPJ FLEXIBLE ELECTRONICS, vol. 6, no. 1, 25 August 2022 (2022-08-25), XP093104504, Retrieved from the Internet <URL:https://www.nature.com/articles/s41528-022-00211-6> [retrieved on 20231122], DOI: 10.1038/s41528-022-00211-6
- ALAWIEH HUSSEIN ET AL: "Towards Point-of-Care Heart Failure Diagnostic Platforms: BNP and NT-proBNP Biosensors", SENSORS, vol. 19, no. 22, 16 November 2019 (2019-11-16), pages 5003, XP055920264, Retrieved from the Internet <URL:https://www.ncbi.nlm.nih.gov/pmc/articles/PMC6891483/pdf/sensors-19-05003.pdf> [retrieved on 20231123], DOI: 10.3390/s19225003

## Description

### FIELD OF INVENTION

The present invention relates to an organic electrochemical sensor.

### BACKGROUND OF INVENTION

Heart failure is a chronic life-threatening disease needing lifelong management with 30% of patients being readmitted within 30 days after discharge. For most patients, the goal of therapy and pharmaceuticals is to improve patients' quality of life, slow down disease progression and avoid acute life-threatening events during which hospitalization becomes necessary. Optimizing pharmaceutical dosing is patient specific; it requires titration to balance therapeutic efficacy with minimal side effects. While efforts are being made to reduce hospitalizations and readmissions, these are focused on monitoring surrogate and late indicators of heart failure acute events such as weight and patient symptoms, as opposed to frequent blood testing which can provide an early indication that an adjustment to patient medication dosage is required.

However, known detection method and device are not satisfactory.

Indeed, current blood testing exclusively relies on clinical chemistry in laboratories or point-of-care devices that can only be used in professional healthcare setups. There is currently no solution that provides for home monitoring of all the key blood biomarkers for assessing disease evolution in heart failure. The patient thus needs to go regularly to the hospital or the doctor's office to be monitored which may be difficult and leads to a lack of proper monitoring. In the worst case, the patient is taken to the hospital because the symptoms are present which implies that it is too late to detect heart failure aggravation without or with small aftereffects.

The same issue arises in many other chronic diseases.

Organic electrochemical transistors (OECTs) have been recently developed. An OECT basically comprises a source electrode and a drain electrode connected by a channel and a gate electrode. By functionalizing the gate or the channel of the OECTs, it is possible to use them as biosensors, *i.e.*, it allows detection and quantification of analytes in a sample. For example, the European patent application EP 3 045 902 discloses an electrolyte-gate sensors with functionalized PEDOT channel regions. However, known OECTs merely allow to detect only one analyte at a time. For example, Lin Baojun et Al. ("Flexible organic integrated electronics for self-powered multiplexed ocular monitoring", NPJ FLEXIBLE ELECTRONICS, vol. 6, no. 1, 25 August 2022) discloses an OECT integrated in contact lens to only monitor glucose level. Alawieh Hussein et Al. ("Towards Point-of-Care Heart Failure Diagnostic Platforms: BNP and NT-proBNP Biosensors", SENSORS, vol. 19, no. 22, 16 November 2019) as well as the United-States patent US 6 241 863 disclose a single OECT configured for a specific detection of one analyte. Therefore, several OECTs with different functionalization are needed to diagnose heart failure which leads to an increase of the manufacturing costs and a complex use for the patient. For example, Demuru Silvia et Al. ("Real-Time Multi-Ion Detection in the Sweat Concentration Range Enabled by Flexible, Printed, and Microfluidics-Integrated Organic Transistor Arrays", ADVANCED MATERIALS TECHNOLOGIES, vol. 5, no. 10, 16 August 2020) discloses a system composed of four individual OECTs.

Thus, there is a need for a device suitable for the detection and measurement of a plurality analytes in a single sample with high sensibility and high sensitivity in order to monitor, for example, heart failure. The device needs to be simple in order to be used by a patient, as well as compact and requiring reduced manufacturing costs.

A purpose of this invention is therefore to provide an organic electrochemical sensor solving one or some of the drawbacks of known devices, in particular for the measure and monitoring of heart failure analytes. The sensor comprises a plurality of source electrodes and drain electrodes connected by channels and a plurality of gate electrodes. Some of the channels and gates are functionalized so that different analytes can be measured in an unprepared physiological sample such as a single blood drop.

### SUMMARY

To this aim, the invention relates to an organic electrochemical sensor for the detection of analytes in a physiological sample.

The sensor according to the present invention is defined in claim 1.

In an embodiment, the channels (C₂, C₃, C₄) and gates (G₂, G₃, G₄) satisfy said three conditions. In other words, the sensor comprises a selective ion recognition layer and a faradaic recognition layer and an affinity recognition layer.

Indeed, the sensor allows to measure four analytes-in a single sample, these analytes being of very different chemical natures. The measure of at least two analytes allows for instance to monitor heart failure evolution without requiring multiple sampling and/or analysis. Remote blood testing and direct measure of biomarkers indicative of deterioration in health status can provide data for sound decision-making concerning medication titration and management. Such monitoring allows practitioners to react swiftly to patient health status changes without requiring an office visit and before the patient may need an emergency room visit or hospital admission.

The use of functionalized channel and gate allows the detection of analytes in an unprepared sample. Indeed, the functionalization allows to increase the sensitivity to a target. Therefore, the target is detectable in a raw sample which was not prepared, for example, to increase the concentration of said target. The sensor of the invention is thus easy to use.

Moreover, the unfunctionalized channel and gate allow reference measurements which are thus the same for the different functionalized gates and channels. The reference measurements advantageously eliminate the variations of the offset between the organic electrochemical transistors resulting in an optimized sensitivity.

According to an advantageous aspect of the invention, the faradaic recognition layer is an enzymatic recognition layer allowing recognition of the specific analyte by enzymatic reaction with the sample, preferably the enzymatic recognition layer is configured to detect creatinine. Here, the enzymatic reaction includes a reduction or oxidation step.

The detection and measure of creatinine allows to detect and evaluate the risk of kidney impairment or kidney failure, due to its comorbid nature in heart failure events as well as potential side effects of diuretics on patient kidneys.

According to an advantageous aspect of the invention, the affinity recognition layer comprises at least one of the following: specific antibodies, aptamers, nanobodies or molecularly imprinted polymers.

The affinity recognition layer is configured to detect NT-pro-BNP.

The detection and measure of NT-pro-BNP relates to the physiological state of the heart muscle and its ability to pump blood. This analyte thus allows to evaluate the probability of heart failure event.

According to claim 1, at least one channel comprises an ion recognition layer being configured to detect Potassium ion.

Indeed, a high level of Potassium relates to a decay of elasticity of heart blood vessels which may lead to arrhythmia and could result in a decompensation event. This analyte can thus be used in the detection and monitoring of heart failure.

According to claim 1, at least one channel comprises an ion recognition layer being configured to detect Sodium ion.

The measure of Sodium level allows to monitor the side effects that may be generated by the medications preventing heart failure. Indeed, an excessive Sodium intake is one of the major risk factors for causing complications in patients with the heart failure. Therefore, measuring level of Sodium is complementary to the direct detection of heart failure.

According to an advantageous aspect of the invention, the enzymatic recognition layer is configured to perform a multi-enzyme cascade reaction.

The use of a multi-enzyme cascade reaction allows to increase the sensitivity of the sensor to detect small molecules.

According to an advantageous aspect of the invention, the organic electrochemical sensor further comprises a gate configured for the direct detection of creatine.

According to an advantageous aspect of the invention, the faradaic recognition layer for the detection of creatine comprises creatinase, hence it is an enzymatic recognition layer in this case. It allows to distinguish the native creatine present in the sample from the creatin created by the multi-enzyme cascade reaction.

The mutualization allows to reduce the total number of electrodes in the sensor thereby increasing the compactness of the sensor and decreasing the manufacturing costs. It also decreases the complexity of the sensor, which improves its robustness and simplifies the quality control.

The organic electrochemical sensor may further comprise one gate comprising an enzymatic recognition layer configured to detect creatine.

According to an advantageous aspect of the invention, the size of the organic electrochemical sensor is lower than 5 cm².

This allows to measure the analytes in a small volume of sample thanks to the proximity between the organic electrochemical transistors.

The invention also relates to an electronic device comprising an organic electrochemical sensor as described above.

Finally, the invention also relates to a method for detecting analytes in a physiological sample according to claim 9.

It has to be noted that the order of measurement for all OECTs of step c) is indifferent. Similarly, the order of measurement for all OECTs of step d) is indifferent. Last, the order of steps c) and d) is indifferent. Actually, all required OECTs are used for a measurement in any order.

### DEFINITIONS

In the present invention, the following terms have the following meanings:

"Detection" refers to the identification of a biological molecule - or analyte - in a sample (qualitative) or to the measurement of the concentration of a biological molecule - or analyte - in a sample (quantitative).

"**Mutualization**" refers to the use of the same electrode by two different OECTs in order to apply a voltage to or measure the current from different electrodes using only one electrical connection. In particular, an electrode may be a drain for a specific OECT and a source for another specific OECT, the same electrode being used sequentially in both OECTs.

"**Unfunctionalized**" refers to a channel or a gate that does not comprise any specific recognition layer. An unfunctionalized surface may be uncoated or coated with non-specific chemicals.

"**Voltage applied to a channel**" refers to an electric potential difference applied between the drain and the source connected by said channel.

"**Voltage applied to a gate**" refers to an electric potential difference applied between the gate and a source.

### DETAILED DESCRIPTION

The following detailed description will be better understood when read in conjunction with the drawings. For the purpose of illustrating, the organic electrochemical sensor is shown in the preferred embodiments. It should be understood, however that the application is not limited to the precise arrangements, structures, features, embodiments, and aspect shown. The drawings are not drawn to scale and are not intended to limit the scope of the claims to the embodiments depicted.

This invention relates to an organic electrochemical sensor comprising components which, in some arrangements, define at least three organic electrochemical transistors (OECT). An organic electrochemical transistor is known to comprise a source electrode and a drain electrode connected by an organic conductive channel, and a gate electrode. The conductive channel may be a n-type or p-type polymer such as PEDOT:PSS. The gate electrode may be organic (for example comprising PEDOT:PSS). Source and drain electrodes preferably comprise inorganic conductive compounds.

It must be noted that the source electrode, drain electrode and gate electrode may also be simply named the source, drain and gate in the following description.

The organic electrochemical sensor of the invention comprises at least three gates (G₁, G₂, G₃, G₄) and at least four sets of electrodes. Each set of electrodes comprises a source electrode (S₁, S₂, S₃, S₄) and a drain electrode (D₁, D₂, D₃, D₄) connected by a conductive channel (C₁, C₂, C₃, C₄). The electrodes may be coated with an intermediate layer. For example, the intermediate layer is chosen in order to improve the adhesion between the surface of the electrode and the functional layer described hereafter.

At least two of the channels (C₂, C₃, C₄) and two of the gates (G₂, G₃, G₄) are functionalized, *i.e.*, they comprise a recognition layer on their surface. The recognition layer is selected from
i. selective ion recognition layer;
ii. faradaic recognition layer allowing recognition of a specific analyte by the reduction or oxidation of said analyte; or
iii. an affinity recognition layer.

In the invention, the channels (C₂, C₃, C₄) and gates (G₂, G₃, G₄) comprise the three types of recognition layers.

Such functionalization allows for specific sensing (such as detection, concentration measurement...) of biological molecules (analytes) in a physiological sample deposited on the sensor for analysis without need of any preparation of the sample. Indeed, the gate electrode establishes electrical connection with channel through an electrolyte medium. The electrolyte medium is the sample under analysis, in which the analyte is looked for. The sample thus behaves like an ion reservoir. When the sample is deposited on the gate (G₂, G₃, G₄) or channel (C₂, C₃, C₄) comprising a recognition layer, ions from reservoir are injected in or withdrawn from the gate (G₂, G₃, G₄) (respectively the channel (C₂, C₃, C₄)) thanks to the reaction or interaction between the recognition layer and the target analyte. This therefore leads to a change in the electronic charge density of the channel and thus in the drain current which may be measured by a usual electronic device. The measured current is thus finally an indication of presence of the target analyte interacting with the corresponding recognition layer.

Moreover, at least one of the channels C₁ and at least one of the gates G₁ are unfunctionalized. By "unfunctionalized", it is meant that the surface of the channel C₁ and the gate G₁ does not comprise any specific recognition layer. Nevertheless, the unfunctionalized surface may be coated with non-specific chemicals. For instance, a gate G₁ may comprise neutral protein(s), such as for example Bovine serum albumin (BSA), Prionex^{™} reagent, casein, newborn calf serum (NBCS), or 2-mercaptoethanol, to avoid any specific adsorption on the gate G₁. The unfunctionalized gate G₁ and channel C₁ thus provide with a normalized (reference) signal, *i.e.*, not sensitive to any specific biological molecules. This reference signal allows to eliminate noise and improve precision of the sensor. Moreover, the reference measurements advantageously eliminate the variations of the offset between the organic electrochemical transistors resulting in an optimized sensitivity. Preferably, the unfunctionalized surfaces comprise the same intermediate layer - except the functionalization - than the surfaces comprising the recognition layer so that the measurement between the functionalized and unfunctionalized surfaces can be directly compared.

The sensor of the invention is thus configured so that any set of electrodes (one source (S₁, S₂, S₃, S₄) and one drain (D₁, D₂, D₃, D₄)), the channel (C₁, C₂, C₃, C₄) connected to said set of electrodes and any gate electrode (G₁, G₂, G₃, G₄) form an organic electrochemical transistor (OECT). If both the channel (C₁) and the gate (G₁) are unfunctionalized, the OECT enables a reference measure of the sample. If either said channel (C₂, C₃, C₄) or said gate (G₂, G₃, G₄) comprises a recognition layer, then the OECT is designed to measure a signal associated to the target of the recognition layer. Anyway, in one OECT, one gate or one channel is unfunctionalized - optionally, both are unfunctionalized. The number of OECTs that may be formed is at least equal to the number of functionalized surfaces, with optionally at least one additional OECT for reference measure. Therefore, at least three OECTs are comprised in the sensor of the invention.

It has to be noted that the sensor of the invention is not a simple combination of OECTs comprising each a functionalized gate or a functionalized channel. Indeed, in the invention, the number of functionalized and unfunctionalized gates (G₁, G₂, G₃, G₄) and channels (C₁, C₂, C₃, C₄) are reduced so that to optimize the compactness of the device. In other words, the different organic electrochemical transistors that can be formed share the unfunctionalized gate G₁ and channel C₁ in order to reduce the total number of electrodes.

The functionalized surfaces of the organic electrochemical sensor of the invention are:
- at least two channel (C₂, C₃, C₄) surfaces comprising a selective ion recognition layer;
- at least one gate (G₂, G₃, G₄) surface comprising a faradaic recognition layer;
- at least one gate (G₂, G₃, G₄) surface comprising an affinity recognition layer.

This configuration allows to achieve an optimal combination of a channel with high transconductance and a gate with high capacitance on a minimum sensor surface. Such a combination can be obtained with a low-cost screen printing and ink-jet printing manufacture technologies.

The recognition layer may completely cover the corresponding gate (G₂, G₃, G₄) or corresponding channel (C₂, C₃, C₄).

The selective ion recognition layer allows to select one specific ion. The ions are Sodium (Na⁺) and Potassium (K⁺).

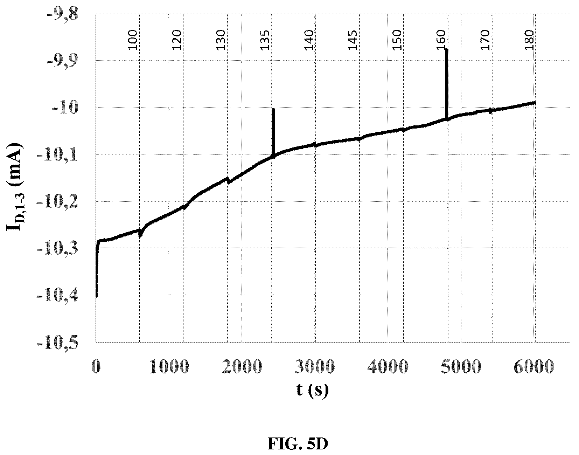

The measurement of Sodium ion concentration in blood is challenging because it has a very narrow range of concentrations with very high values (normal values ranging from 135 to 145 mEq/L in human blood - see figure 5D). Thus, both the sensitivity resolution and the saturation point must be high. Covering a channel (C₂, C₃, C₄) with a Sodium ion recognition layer thus allows a linear variation of the response of the OECT with the concentration of Sodium from 100 to 180 mEq/L for instance. Measuring the Sodium concentration allows to monitor the side effects that may be generated by the medications preventing, for example, heart failure. Therefore, measuring the level of Sodium is complementary to the detection, prevention and monitoring of heart failure.

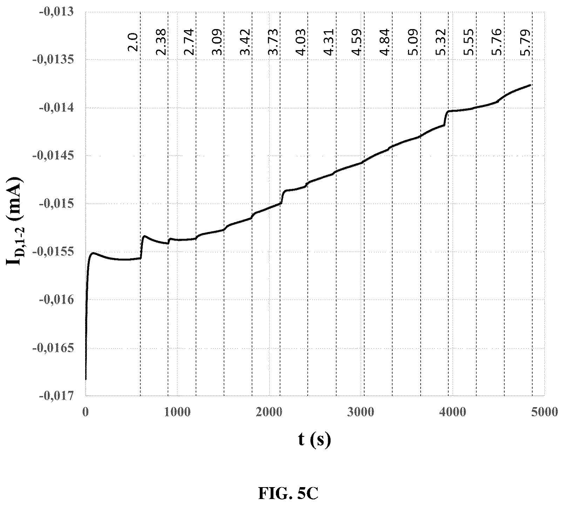

The measurement of Potassium ion concentration is also challenging since its concentration in the blood is low in comparison with the other electrolytes (normal values ranging from 3.5 to 5.1 mEq/L in a whole blood media - see figure 5C). The selectivity must thus be high. Covering a channel (C₂, C₃, C₄) with a Potassium ion recognition layer thus allows a linear variation of the response of the OECT with the concentration of Potassium from 2.5 to 6 mEq/L for instance. A high level of Potassium relates to a decay of elasticity of heart blood vessels which may lead to arrhythmia and could result in a decompensation event. This analyte is thus useful in the detection, prevention and monitoring of heart failure.

By faradaic recognition layer, it is meant a layer in which an analyte undergoes either reduction or oxidation. The electric current thus created modifies OECT electronic properties and enable electric measurement.

The faradaic recognition layer is an enzymatic recognition layer, in which a single enzyme or several enzymes - optionally working in a multi-enzyme cascade reaction - are used to assist reduction or oxidation of the analyte. The enzymatic recognition layer allows a recognition of a specific analyte by enzymatic reaction with the sample. More precisely, it allows for the recognition of hydrolysable molecules. The enzymatic recognition layer is configured to detect creatinine. To do so, the enzymatic recognition layer may comprise creatininase or creatinine amidinohydrolase. The creatininase (also named creatinine deaminase) and creatinine amidinohydrolase are enzymes which hydrolyze creatinine into creatine. The measure of creatinine in blood allows to evaluate the risk of kidney failure, due to its comorbid nature. However, the detection of creatinine through the direct interaction with the recognition layer is challenging due to absence of reliable recognition elements. Therefore, the enzymatic recognition layer may be configured to perform a multi-enzyme cascade reaction. For example, using a multi-enzyme cascade of three or four enzymes allows a degradation of the creatinine in creatine, the first step of the multi-enzyme cascade being hydrolysis of creatinine into creatine.

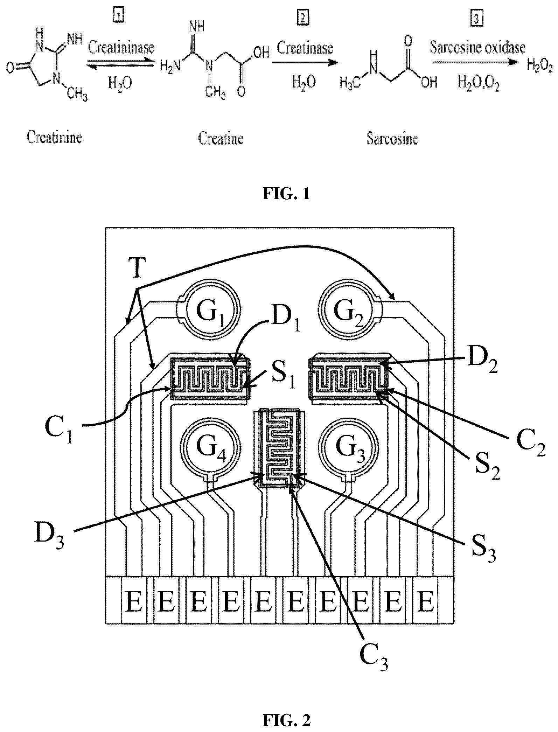

An example of multi-enzyme cascade is represented in figure 1. This multi-enzyme cascade allows to transform existing creatinine to H₂O₂, which is then quantified electrochemically - with an enzyme - to get the signal, which corresponds to the creatinine concentration. The multi-enzyme cascade may comprise a mediator. The role of the mediator is to improve the transfer of any electron formed as a result of the electrochemical reaction towards an electrode of the OECT. The mediator may be selected from the group of 2,2'-Azino-bis(3-ethylbenzothiazoline-6-sulfonic acid); Ferrocene, Ferrocene monocarboxylic acid; Ferri/ferro-cyanide; 2,6-Dichlorophenol-indophenol; Hexaamineruthenium; 1,4-Naphthoquinone; 7-Hydroxy-3H-phenoxazin-3-one (also know as Resorufin); 1,1'-bis(cyanomethyl)-4,4'-bipyridyl (also known as Cyanomethylviologen); 1,1'-bis(ethyl)-4,4'-bipyridyl (also known as Ethylviologen); Riboflavin 5'-monophosphate; 1,1'-ethylene-2,2'-bipyridyl (also known as Diquat); 1,1'-trimethylene-2,2'-bipyridyl (also known as Triquat); or 4,4'-Bipyridinium-1,1'-bis(2-ethylsulfonate). A preferred mediator is Ferrocene. In the example of figure 1, the enzyme is HRP (horse radish peroxidase).

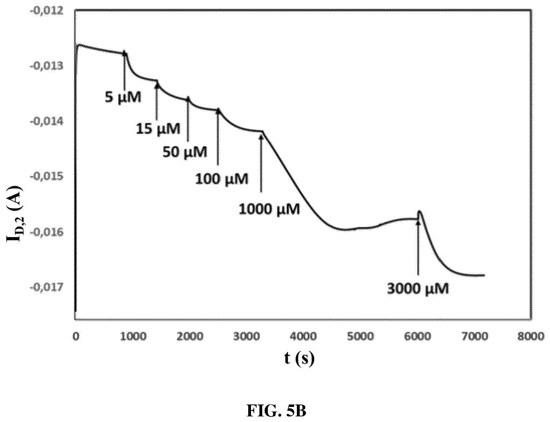

A difficulty when using a multi-enzyme cascade reaction is to distinguish between the native creatine (present in the blood sample) and the creatine created during the multi-enzyme cascade that has to be measured to determine the concentration of creatinine. To overcome this difficulty, a first solution is to measure only the creatine created by the degradation of the creatinine. This solution may be implemented by two methods. Firstly, degrading the native creatine from the blood in the inlet of the sample deposition area, before it reaches the enzymatic recognition layer. To do so, the enzymatic recognition layer for the detection of creatine comprises creatinase for instance. Secondly, including, on the sensor, an additional reference electrode. Preferably, the additional reference electrode comprises the same intermediate layer - except the enzyme allowing hydrolyzis of creatinine into creatine - than the electrode comprising the enzymatic recognition layer for detecting creatinine. The difference of signal between the electrode comprising the enzymatic recognition layer and the additional reference electrode thus allows to measure the creatinine issued from the multi-enzyme cascade reaction. Another solution to overcome the difficulty is that one of the gates G₄ is configured to directly detect the native creatine, the concentration of the native creatine is thus subtracted from the total concentration of creatine measured after the multi-enzyme cascade reaction so that to determine the concentration of the creatine generated by the multi-enzyme cascade reaction. The sensor of the invention allows to detect the creatinine with a concentration ranging from 0 to 3000 µM in a whole blood media - see figure 5B.

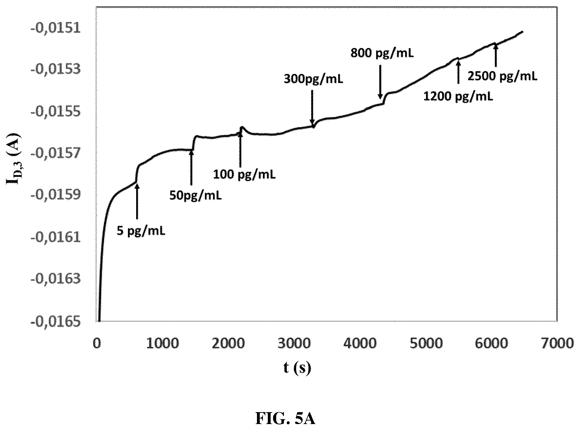

The affinity recognition layer allows a recognition of a specific analyte in the sample by affinity. More precisely, it allows for the recognition of non-hydrolysable molecules using specific antibody (antibody fragments, antibody dendrimer conjugates, nanobodies) or aptamers interaction or engineered binding proteins on the surface of the electrode or nanobody interaction or molecularly imprinted polymers. The affinity recognition layer is configured to recognize the N-terminal fragment of the B-type natriuretic peptide (NT-pro-BNP). The detection of NT-pro-BNP is challenging because it is present at a very low level in blood (between less than 100 pg/mL to 450 pg/mL for healthy patients and could go up to 20,000 pg/mL in a very sick patient). Using specific antibody interaction for the recognition of NT-pro-BNP advantageously generates a linear response of the OECT with the concentration of the peptide from 0 to 300 pg/mL for instance. The combination of two linear responses allow to reach a detection from 5 pg/mL to 30,000 pg/mL - see figure 5A. A NT-pro-BNP level larger than 450 pg/mL reflects an inability for the heart muscle to pump blood.

Electrical connections E may be arranged on the sensor to facilitate the application of a voltage to or the measure of a current from the different electrodes.

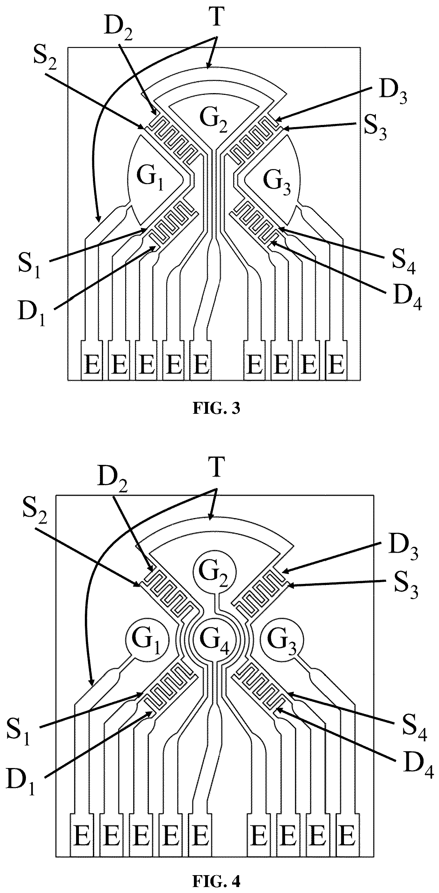

Mutualization (or sharing) of the source (S₁, S₂, S₃, S₄) and drain (D₁, D₂, D₃, D₄) electrodes means that the source (S₁, S₂, S₃, S₄) or drain (D₁, D₂, D₃, D₄) electrodes are electrically connected so that the application of a voltage to or the measure of a current from the mutualized electrodes may be performed from the same electrical connection E. According to the present invention and shown in the figures 3 and 4, two pairs of sources ((S₁, S₂) and (S₃, S₄)) and two drain (D₂, D₃) electrodes are mutualized. Drain and source electrodes may also be interdigitated. The mutualization allows to reduce the total numbers of electrodes in the sensor (from 8 to 5 in this case) thereby increasing the compactness of the sensor and decreasing the manufacturing costs. This also allows to work with small user's samples. It also decreases the complexity of the sensor, which improves its robustness and simplifies its quality control.

Figure 2 shows an organic electrochemical sensor comprising 4 gates (G₁, G₂, G₃, G₄) and 3 channels (C₁, C₂, C₃) connecting 3 drains (D₁, D₂, D₃) to 3 sources (S₁, S₂, S₃) - drains and sources are interdigitated electrodes here. In this embodiment, one channel C₂ comprises the Sodium ion recognition layer, another channel C₃ comprises the Potassium ion recognition layer, one gate G₂ comprises the affinity recognition layer configured to recognize NT-pro-BNP and another gate G₃ comprises the enzymatic recognition layer configured to detect creatinine. The other channel C₁ and the two other gates (G₁, G₄) are unfunctionalized.

Figure 3 shows an organic electrochemical sensor comprising 3 gates (G₁, G₂, G₃) and 4 channels (C₁, C₂, C₃, C₄) (not shown but located above the drain and source pairs) connecting 4 drains (D₁, D₂, D₃, D₄) to 4 sources (S₁, S₂, S₃, S₄) - drains and sources are interdigitated electrodes here. As explained above, several source (S₁, S₂ and S₃, S₄) and drain (D₂, D₃) electrodes are mutualized. The channel geometry of this embodiment allows to increase the output intensity and signal to noise ratio. In this embodiment, one channel C₂ comprises the Sodium ion recognition layer, another channel C₃ comprises the Potassium ion recognition layer, one gate G₂ comprises the affinity recognition layer configured to recognize NT-pro-BNP and another gate G₃ comprises the enzymatic recognition layer configured to detect creatinine. The other two channels (C₁, C₄) and the remaining gate G₁ are unfunctionalized.

Figure 4 shows an organic electrochemical sensor comprising 4 gates (G₁, G₂, G₃, G₄) and 4 channels (C₁, C₂, C₃, C₄) (not shown, but located above the drain and source pairs) connecting 4 drains (D₁, D₂, D₃, D₄) to 4 sources (S₁, S₂, S₃, S₄) - drains and sources are interdigitated electrodes here. As explained above, several source (S₁, S₂ and S₃, S₄) and drain (D₂, D₃) electrodes are mutualized. One of the gates G₄ has a central position surrounded by the channels. This geometry allows to increase the sensitivity. Compared to the previous embodiment, in this embodiment, two gates (G₁, G₄) are unfunctionalized.

This last embodiment is advantageous. Indeed, the optimized geometry allows to decrease the surface area, which leads to a smaller user's sample volume needed and makes the sensor suitable for a use with capillary blood samples. The position of the active sensing elements thus allows the rapid and specific detection of multiple analytes in only one single sample. Finally, this geometry also eliminates the need of microfluidics implementation, and thus decreases the manufacturing complexity and cost.

In all of the above-described embodiments, the sensor may comprise successive layers described hereafter.

An insulating substrate may be provided as a support. The substrate may be selected from plastic, paper and glass. The substrate is preferably a flexible polymer substrate, such as polyethylene terephthalate (PET); polyimide (Kapton^{™}) or paper substrate.

An electric layer may be deposited on the substrate. The electric layer comprises sources (S₁, S₂, S₃, S₄), drains (D₁, D₂, D₃, D₄), gates (G₁, G₂, G₃, G₄) and channels (C₁, C₂, C₃, C₄) as well as electrical connections E and the conductive tracks T between them. Sources (S₁, S₂, S₃, S₄), drains (D₁, D₂, D₃, D₄) and gates (G₁, G₂, G₃, G₄) are metallic or non-metallic electrodes deposited on the substrate. Gold and its alloys are particularly suitable for electrodes. The electric layer may also be a sandwich-like layer such as Ag-C-Ag/AgCl; Ag-Ag/AgCl; or Ag-C. Source (S₁, S₂, S₃, S₄) and drain (D₁, D₂, D₃, D₄) electrodes are preferably printed using silver and carbon screen-printing inks, which consist of a mixture of silver or carbon with different polymers for an optimal fluidity. This results in electrodes comprising an organic non-conductive matrix and conductive inorganic components. The ink may be a protective conductive ink to protect electrodes from oxidation by the sample.

The channel (C₂, C₃, C₄) may comprise a first intermediate conductive layer to connect the source (S₂, S₃, S₄) and drain (D₂, D₃, D₄). This intermediate layer ensures an efficient operation of the ion sensor and thus an improvement of the signal and the sensitivity of the sensor. The ion selective recognition layer may then be deposited on the intermediate conductive layer. The ion selective recognition layer may comprise an ionophore (*i.e.*, selective towards Sodium or Potassium).

The gate (G₂, G₃, G₄) may comprise a covalent self-assembled monolayer. The recognition layer may then be deposited on the monolayer.

In the case of enzymatic recognition layer, the enzymes are either chemically cross-linked to a mediator solution before the deposition on the monolayer or non-covalently bounded by drying the enzymes on a surface which is then linked to a cellulose membrane to be put on the surface of the electrode.

The sensor may further comprise a filtering membrane over the recognition layer of the gate (G₂, G₃, G₄). The membrane may also cover the whole electrode and recognition layer. Such membrane works as a filter between deposited sample and electrodes. In particular, such membrane may be useful with unprepared blood samples, so that only plasma can get in contact with recognition layer which is thus less polluted leading to an improved response and signal-to-noise ratio of the OECT. Suitable selective membranes are commercially available, for instance LF1, MF1, VF1 and VF2 from Whatman International Ltd. (Maidstone, England).

Finally, a dielectric ink is printed on the surface of the sensor leaving only the channels and functional electrode's surfaces uncovered.

All the layers above may be deposited by any suitable physical or chemical method including inkjet printing, drop casting, roll-to-roll, screen printing, spin coating and vacuum physical and chemical deposition.

In one embodiment, the size of the organic electrochemical sensor is lower than 5 cm², preferably lower than 3 cm², preferably lower than 2 cm². By size, it is meant the area of a convex shape encompassing all gates (G₁, G₂, G₃, G₄) and OECT arrangements of the sensor. This size corresponds to the area on which a sample should be spread to be analyzed correctly. This advantageously allows to measure the analytes in a small volume of sample thanks to the proximity of the organic electrochemical transistors. For example, the volume of the sample may be about 100 µL (500 µm thick layer of sample over 2 cm² for instance) or even lower than 40 µL.

Electrodes may have various dimensions, depending on size and precision constraints. Source (S₁, S₂, S₃, S₄) and drain (D₁, D₂, D₃, D₄) electrodes may have lateral dimensions (length and width) in the range of 100 nm to 2 cm, preferably in the range of 1 µm to 1.5 cm, more preferably in the range of 50 µm to 1 cm. Gate electrode (G₁, G₂, G₃, G₄) may have lateral dimensions (length and width) in the range of 100 nm to 5 cm, preferably in the range of 1 µm to 3 cm, more preferably in the range of 50 µm to 2 cm.

Surface of each electrode is typically in the range of 1 mm² to 100 mm². Surface of channel is typically in the range of 0.1 mm² to 10 mm².

Organic electrochemical sensor of the invention is particularly suitable for testing blood, saliva, urine and other biological fluid. In particular it is suitable to monitor heart failure in a blood sample.

The invention also relates to an electronic device comprising an organic electrochemical sensor according to any embodiment described above. The electronic device further comprises all electrical elements required to impose voltage to electrodes, measure current flows, acquire and analyze signals.

The invention also relates to a method for detecting analytes in a physiological sample according to claim 9. Any embodiment of the organic electrochemical sensor described hereabove is suitable for this use. The first step of the method is to provide the organic electrochemical sensor of the invention.

A physiological sample is disposed on the channels and the at least three gates (G₁, G₂, G₃) of the sensor.

For each organic electrochemical transistor comprising the enzymatic recognition layer or the affinity recognition layer:
- voltages are respectively applied to the unfunctionalized channel C₁ (*i.e.*, the voltage is applied to the drain D₁ electrode while the source S₁ electrode is grounded) and to the unfunctionalized gate G_{1;} and to the unfunctionalized channel C₁ and to the functionalized gate (G₂, G₃),
- an output is then measured, the output being a change of a value of drain current.

The order of application of voltages is indifferent: voltage may be applied first to the unfunctionalized channel C₁ and to the unfunctionalized gate G₁. Alternatively, voltage may be applied first to the unfunctionalized channel C₁ and to the functionalized gate (G₂, G₃).

For each organic electrochemical transistor comprising the selective ion recognition layer:
- voltages are respectively applied to the unfunctionalized channel C₁ and to the unfunctionalized gate G₁ and to the functionalized channel C₂ and to the unfunctionalized gate G₁,
- an output is measured, the output being a difference of value of current between the two drain (D₁, D₂) electrodes.

Similarly, the order of application of voltages is indifferent: voltage may be applied first to the unfunctionalized channel C₁ and to the unfunctionalized gate G₁. Alternatively, voltage may be applied first to the functionalized channel C₂ and to the unfunctionalized gate G₁.

In addition, the order of measurement of the OECTs comprising the enzymatic recognition layer, the affinity recognition layer or the selective ion recognition layer is indifferent.

The voltages applied to the channels and the gates are preferably applied consecutively in a short period of time. The consecutive voltages do not necessarily have the same voltage value: their intensities are adapted for each OECT.

As described hereabove, the change of a value of drain current is a function of the concentration of the analyte detected by the recognition layer.

The sensor is preferably a single use sensor so that the detection of each analyte detectable with the different OECTs of the sensor may be performed once.

The method for detecting analytes may further comprise, before disposing the sample, a calibration stage comprising the steps of:
- Disposing, on the channels (C₁, C₂) and the at least three gates (G₁, G₂, G₃), a calibration sample comprising a determined concentration of analyte detectable by at least one of the organic electrochemical transistors of the organic electrochemical sensor,
- For each organic electrochemical transistor comprising the enzymatic recognition layer or the affinity recognition layer:
   i. voltages are respectively applied to the unfunctionalized channel C₁ (*i.e.*, the voltage is applied to the drain D₁ electrode while the source S₁ electrode is grounded), to the unfunctionalized gate G₁; and to the unfunctionalized channel C₁ and to the functionalized gate (G₂, G₃),
   ii. a first output is then measured, the output being a change of a value of drain current.
- For each organic electrochemical transistor comprising the selective ion recognition layer:
   i. voltages are respectively applied to the unfunctionalized channel C₁, to the unfunctionalized gate G₁; and to the functionalized channel C₂ and to the unfunctionalized gate G₁,
   ii. a second output is then measured, the output being a difference of value of current between the two drain (D₁, D₂) electrodes.
- Increasing the determined concentration of the analyte in the sample,
- Repeating at least one time the steps i. to ii. for each organic electrochemical transistor,
- Determining the correspondence between each of the first and the second output and the concentration of the analyte, and
- Optionally, rinsing the organic electrochemical sensor to that it can be used to detect the analyte in an unknown sample.

As previously, the voltages applied to the channels and the gates are preferably applied consecutively in a short period of time. The consecutive voltages do not necessarily have the same voltage value: their intensities are adapted for each OECT.

### BRIEF DESCRIPTION OF THE DRAWINGS

**Figure 1** shows the enzymatic chain reaction for the detection of creatinine. In a first step, creatinine is transformed by creatininase (also named creatinine aminohydrolase) into creatine. In a second step, creatine is transformed by creatinase (also named creatine aminohydrolase) into sarcosine - and urea, not shown. In a third step, sarcosine is transformed by Sarcosine Oxydase (SOx) into hydrogen peroxide H₂O₂ - and formaldehyde and glycine, not shown.
**Figure 2** shows an organic electrochemical sensor comprising 4 gates and 3 channels connecting 3 drains to 3 sources.
**Figure 3** shows an organic electrochemical sensor comprising 3 gates and 4 channels connecting 4 drains to 4 sources. The drain and source electrodes are mutualized.
**Figure 4** shows an organic electrochemical sensor comprising 4 gates and 4 channels connecting 4 drains to 4 sources. One of the gates has a central position encircled by the channels. The drain and source electrodes are mutualized.
**Figure 5A-D** are a combination of graphs showing the evolution in a whole blood medium of the current measured with the organic electrochemical sensor of **figure 4****.** **Fig. 5A** shows the current I_{D,3} (in Ampere) as a function of time t (in seconds) while the concentration of NT-pro-BNP is regularly increased from 0 pg/mL to 2500 pg/mL (identified by arrows). **Fig. 5B** shows the current I_{D,2} (in Ampere) as a function of time t (in seconds) while the concentration of creatinine is regularly increased from 5 µM to 3000 µM (identified by arrows). **Fig. 5C** shows the current difference I_{D,1-2} (in milli-Ampere) as a function of time t (in seconds) while the concentration of Potassium ion is regularly increased from 2.0 mEq/L to 5.96 mEq/L (identified by columns). **Fig. 5D** shows the current difference I_{D,1-3} (in milli-Ampere) as a function of time t (in seconds) while the concentration of Sodium ion is regularly increased from 100 mEq/L to 180 mEq/L (identified by columns).

### EXAMPLES

The present invention is further illustrated by the following examples.

In these examples, the organic electrochemical sensor comprises, as represented in figure 4:
- one channel C₂, connecting the source S₂ and the drain D₂, comprising a selective Potassium ion recognition layer;
- one channel C₃, connecting the source S₃ and the drain D₃, comprising a selective Sodium ion recognition layer;
- one gate G₂ comprising an enzymatic recognition layer configured to detect creatinine;
- one gate G₃ comprising an affinity recognition layer configured to detect NT-pro-BNP;
- one gate G₄ comprising an enzymatic recognition layer configured to detect creatin;
- two unfunctionalized channel (C₁, C₄), respectively connecting the source S₁ and the drain D₁ and connecting the source S₄ and the drain D₄;
- two unfunctionalized gates (G₁, G₄);
- four sources (S₁, S₂, S₃, S₄) arranged in two mutualized pairs of sources; and
- four drains (D₁, D₂, D₃, D₄) comprising one mutualized pair of drains.

The sensor preparation is the following.

A flexible PET polymer substrate is provided as a support. The substrate is cleaned, to remove any dust and organic contamination that may be present on its surface.

The electric layer is a sandwich-like Ag-C or Ag-Ag/AgCl layer.

For the channels (C₁, C₂, C₃, C₄), the first intermediate conductive layer is a poly(3,4-ethylenedioxythiophene) polystyrene sulfonate (PEDOT:PSS) based layer. The PEDOT:PSS based layer is covered by a sodium polystyrene sulfonate (PSS:Na) layer for channels (C₂, C₃) comprising an ion recognition layer. This first intermediate conductive layer is thermally cross-linked to form a solid layer.

The ion selective recognition layer is then deposited on the intermediate conductive layer of two channels (C₂, C₃). A solvent from the ion selective recognition layer is then left to evaporate to form a solid film.

The enzymatic recognition layer is a mixture comprising 4 enzymes (Creatinine hydrolase CI, creatine amidinohydrolase CII, Sarcosine Oxidase SOx and HRP + Ferrocene mediator) that is deposited in a non-covalent way on the surface of the gate G₂ electrode. The electrode is then dried at room temperature for 30 minutes or dried in the oven (37°C) during 15 minutes. Finally, a thin layer of sulfonated tetrafluoroethylene-based fluoropolymer-copolymer (Nafion^{™}) is deposited and dried to block interferences between the sample and the electrode. For the additional reference electrode, a mixture of only the 3 enzymes (CI, SOx and HRP) is deposited.

The affinity recognition layer of this example sensor uses biotinylated capture antibodies. Therefore, a layer of chitosan is deposited on the surface of the gate G₃ electrode and dried at room temperature. The amine functions of the chitosan film are activated with glutaraldehyde during one hour before depositing A/G protein. The electrode is then rinsed with PBS to remove unpaired proteins on the chitosan film. The antibodies are then deposited to bind specifically to the A/G proteins. The role of the A/G protein is to orient the antibodies to maximize antigen recognition. The antibodies are incubated during one hour before rinsing and depositing BSA to block the surface and avoid non-specific signal. For the reference electrode only BSA is deposited.

### Example 1: calibration of the organic electrochemical sensor

A voltage of -0.5 V is applied to the unfunctionalized channel C₁ (*i.e.*, the voltage is applied to the drain D₁ electrode while the source S₁ electrode is grounded) while a voltage of 0.15 V is applied to the unfunctionalized gate G₁.

A sample containing controlled concentration of NT-pro-BNP, creatinine, Potassium ion and Sodium ion is brought in contact with the channels (C₁, C₂, C₃, C₄) and the gates (G₁, G₂, G₃, G₄).

The four concentrations are measured successively.

The concentration of creatinine and NT-pro-BNP are measured by applying a voltage of -0.5 V to the unfunctionalized channel C₁ and a voltage of -0.15 V to the functionalized gate (G₂, G₃). A change of a value of drain current I_{D,2} is measured leading to a value representative of the concentration of the creatinine and a change of a value of drain current I_{D,3} is measured leading to a value representative of the concentration of the NT-pro-BNP.

The concentrations of Potassium and Sodium ions are measured by applying a voltage of -0.5 V to the functionalized channel C₂ and C₃ and a voltage of -0.15 V to the unfunctionalized gate G₁. Currents between the drain and the source of each channel are measured. In other word, a difference of value of current I_{D,1-2} is measured between the drain D₂ and the drain D₁ leading to a value representative of the concentration of the Potassium ion and a difference of value of current I_{D,1-3} is measured between the drain D₃ and the drain D₁ leading to a value representative of the concentration of the Sodium ion.

Then sample is replaced with a sample more concentrated in NT-pro-BNP, creatinine, Potassium ion and Sodium ion. The NT-pro-BNP concentration is varied from 0 pg/mL to 2500 pg/mL. The creatinine concentration is varied from 5 µM to 3000 µM. The Potassium concentration is varied from 2.0 mEq/L to 5.96 mEq/L. The Sodium concentration is varied from 100 mEq/L to 180 mEq/L. The concentrations are increased - spiking experiment - by successive increments regularly, identified by arrows or columns on graphs.

Figures 5A-D respectively show the evolution of the currents I_{D,2}, I_{D,3}, I_{D,1-2} and I_{D,1-3} as a function of time, while concentration of analytes is increased progressively.

### Example 2: Measure of concentrations in a whole blood sample using the organic electrochemical sensor

Using the organic electrochemical sensor described above, the concentrations of NT-pro-BNP, creatinine, Potassium ion and Sodium ion are measured in a whole - unprepared - blood sample.

To do so, a sample of blood is brought in contact with the channels (C₁, C₂, C₃, C₄) and the gates (G₁, G₂, G₃, G₄).

The concentration of creatinine and NT-pro-BNP is measured by applying a voltage of -0.5 V to the unfunctionalized channel C₁ and a voltage of -0.15 V to the functionalized gate (G₂, G₃). A change of a value of drain current I_{D,2} of -0.0143 A is measured leading to a concentration of the creatinine of 100 µM and a change of a value of drain current I_{D,3} of -0.0155 A is measured leading to a concentration of NT-pro-BNP of 300 pg/mL.

The concentrations of Potassium and Sodium ions are measured by applying a voltage of -0.5 V to the functionalized channel C₂ and C₃ and a voltage of -0.15 V to the unfunctionalized gate G₁. A difference of value of current I_{D,1-2} of 0.81 mA is measured between the drain D₂ and the drain D₁ leading to a concentration of 4.6 mEq/L for the Potassium ion and a difference of value of current I_{D,1-3} of -10.1 mA is measured between the drain D₃ and the drain D₁ leading to a concentration of 140 mEq/L for the Sodium ion.

## Claims

1. An organic electrochemical sensor for the detection of analytes in a physiological sample, the sensor comprising:
- at least four sets of electrodes, each set of electrodes comprising a source (S₁, S₂, S₃, S₄) electrode and a drain (D₁, D₂, D₃, D₄) electrode, the source and drain electrodes of each set being connected by an organic conductive channel (C₁, C₂, C₃, C₄) and
- at least three gates (G₁, G₂, G₃, G₄),
wherein at least one channel (C₁) and at least one gate (G₁) are unfunctionalized; wherein the channels (C₂, C₃, C₄) and gates (G₂, G₃, G₄) satisfy the following three conditions:
i. at least one channel (C₂, C₃, C₄) or at least one gate (G₂, G₃, G₄) comprises a selective ion recognition layer, preferably a selective alkali ion recognition layer;
ii. at least one channel (C₂, C₃, C₄) or at least one gate (G₂, G₃, G₄) comprises a faradaic recognition layer allowing recognition of a specific analyte by the reduction or oxidation of said analyte at said channel or said gate;
iii. at least one channel (C₂, C₃, C₄) or at least one gate (G₂, G₃, G₄) comprises an affinity recognition layer allowing a recognition of a specific analyte in the sample by affinity;
wherein any set of electrodes, the channel (C₁, C₂, C₃, C₄) connected to said set of electrodes and any gate (G₁, G₂, G₃, G₄) form an organic electrochemical transistor,
**characterized in that** one of the channels (C2) comprises a selective Potassium ion recognition layer, one of the channels (C3) comprises a selective Sodium ion recognition layer, one of the at least three gates (G2) comprises an enzymatic recognition layer configured to detect creatinine; one of the at least three gates (G3) comprises an affinity recognition layer configured to detect NT-pro-BNP; and four of the sources (S1, S2, S3, S4) are arranged in two mutualized pairs of sources and four of the drains (D1, D2, D3, D4) comprise one mutualized pair of drains.

2. The organic electrochemical sensor according to claim 1, wherein the affinity recognition layer comprises at least one of the following: specific antibodies, aptamers, nanobodies or molecularly imprinted polymers.

3. The organic electrochemical sensor according to any one of claims 1 to 2, wherein the enzymatic recognition layer is configured to perform a multi-enzyme cascade reaction.

4. The organic electrochemical sensor according to claim 3, further comprising a gate (G₄) configured for the direct detection of creatine.

5. The organic electrochemical sensor according to claim 3, wherein the enzymatic recognition layer for the detection of creatinine comprises creatininase or creatinine amidinohydrolase.

6. The organic electrochemical sensor according to any one of claims **1** to 5, further comprising one gate comprising an enzymatic recognition layer configured to detect creatine.

7. The organic electrochemical sensor according to any one of claims **1** to 6, wherein the size of the organic electrochemical sensor is lower than 5 cm².

8. Electronic device comprising an organic electrochemical sensor according to any one of claims **1** to 7.

9. A method for detecting analytes in a physiological sample, the method comprising the steps of:
a. Providing the organic electrochemical sensor according to any one of claims **1** to 7,
b. Disposing the sample on the channels (C₁, C₂, C₃, C₄) and the at least three gates (G₁, G₂, G₃, G₄),
c. For each organic electrochemical transistor comprising the enzymatic recognition layer or the affinity recognition layer:
i. voltages are respectively applied to the unfunctionalized channel (C₁) and to the unfunctionalized gate (G₁); and to the unfunctionalized channel (C₁) and to the functionalized gate (G₂, G₃, G₄),
ii. an output is then measured, the output being a change of a value of drain current.
d. For each organic electrochemical transistor comprising the selective ion recognition layer:
i. voltages are respectively applied to the unfunctionalized channel (C₁), to the unfunctionalized gate (G₁); and to the functionalized channel (C₂, C₃, C₄) and to the unfunctionalized gate (G₁),
ii. an output is then measured, the output being a difference of value of current between the two drain (D₁, D₂, D₃, D₄) electrodes.

## Patentansprüche

1. Organischer elektrochemischer Sensor zum Nachweis von Analyten in einer physiologischen Probe, wobei der Sensor Folgendes umfasst:
- mindestens vier Elektrodensätze, wobei jeder Elektrodensatz eine Quellenelektrode (S₁, S₂, S₃, S₄) und eine Abflusselektrode (D₁, D₂, D₃, D₄) umfasst, wobei die Quellen- und Abflusselektroden jedes Satzes durch einen organisch leitenden Kanal (C₁, C₂, C₃, C₄) verbunden sind und
- mindestens drei Tore (G₁, G₂, G₃, G₄),
wobei mindestens ein Kanal (C₁) und mindestens ein Tor (G₁) nicht funktionalisiert sind;
wobei die Kanäle (C₂, C₃, C₄) und Tore (G₂, G₃, G₄) die folgenden drei Bedingungen erfüllen:
i. mindestens ein Kanal (C₂, C₃, C₄) oder mindestens ein Tor (G₂, G₃, G₄) eine selektive Ionenerkennungsschicht umfasst, vorzugsweise eine selektive Alkali-Ionenerkennungsschicht;
ii. mindestens ein Kanal (C₂, C₃, C₄) oder mindestens ein Tor (G₂, G₃, G₄) eine Farada-Erkennungsschicht umfasst, die die Erkennung eines bestimmten Analyten durch die Reduktion oder Oxidation des Analyten an dem Kanal oder Tor ermöglicht;
iii. mindestens ein Kanal (C₂, C₃, C₄) oder mindestens ein Tor (G₂, G₃, G₄) eine Affinitätserkennungsschicht umfasst, die eine Erkennung eines bestimmten Analyten in der Probe durch Affinität ermöglicht;
wobei jeder Elektrodensatz, der Kanal (C₁, C₂, C₃, C₄) verbunden mit dem Elektrodensatz und einem beliebigen Tore (G₁, G₂, G₃, G₄) einen organischen elektrochemischen Transistor bilden,
**dadurch gekennzeichnet, dass** einer der Kanäle (C₂) eine selektive Kaliumionen-Erkennungsschicht umfasst, einer der Kanäle (C₃) eine selektive Natriumionen-Erkennungsschicht umfasst, eines der mindestens drei Tore (G₂) eine enzymatische Erkennungsschicht umfasst, die zum Nachweis von Kreatinin konfiguriert ist; eines der mindestens drei Tore (G₃) umfasst eine Affinitätserkennungsschicht, die zum Nachweis von NT-pro-BNP eingerichtet ist; und vier der Quellen (S₁, S₂, S₃, S₄) in zwei gemeinsam genutzten Quellenpaaren angeordnet sind und vier der Abflüsse (D₁, D₂, D₃, D₄) ein gemeinsam genutztes Abflusspaar umfassen.

2. Organischer elektrochemischer Sensor nach Anspruch 1, wobei die Affinitätserkennungsschicht mindestens einen der folgenden umfasst: spezifische Antikörper, Aptamer, Nanokörper oder molekular geprägte Polymere.

3. Organischer elektrochemischer Sensor nach einem der Ansprüche 1 bis 2, wobei die enzymatische Erkennungsschicht so eingerichtet ist, dass sie eine Multienzym-Kaskadenreaktion durchführt.

4. Organischer elektrochemischer Sensor nach Anspruch 3, ferner umfassend ein Tor (G₄), das für die direkte Detektion von Kreatin eingerichtet ist.

5. Organischer elektrochemischer Sensor nach Anspruch 3, wobei die enzymatische Erkennungsschicht zum Nachweis von Kreatinin Kreatininase oder Kreatininamidinohydrolase umfasst.

6. Organischer elektrochemischer Sensor nach einem der Ansprüche 1 bis 5, ferner umfassend ein Tor, das eine enzymatische Erkennungsschicht umfasst, die zum Erkennen von Kreatin eingerichtet ist.

7. Organischer elektrochemischer Sensor nach einem der Ansprüche 1 bis 6, wobei die Größe des organischen elektrochemischen Sensors kleiner als 5 cm² ist.

8. Elektronische Vorrichtung, umfassend einen organischen elektrochemischen Sensor nach einem der Ansprüche 1 bis 7.

9. Verfahren zum Nachweis von Analyten in einer physiologischen Probe, wobei das Verfahren folgende Schritte umfasst:
a. Bereitstellen des organischen elektrochemischen Sensors nach einem der Ansprüche 1 bis 7,
b. Entsorgen der Probe auf den Kanälen (C₁, C₂, C₃, C₄) und den mindestens drei Toren (G₁, G₂, G₃, G₄),
c. Für jeden organischen elektrochemischen Transistor, der die enzymatische Erkennungsschicht oder die Affinitätserkennungsschicht umfasst:
i. Spannungen an den nicht funktionalisierten Kanal (C₁) beziehungsweise an das nicht funktionalisierte Tor (G₁), und an den nicht funktionalisierten Kanal (C₁) beziehungsweise an das funktionalisierte Tor (G₂, G₃, G₄),
ii. dann wird ein Ausgang gemessen, wobei der Ausgang eine Änderung eines Werts des Ableitstroms ist.
d. Für jeden organischen elektrochemischen Transistor, der die selektive Ionenerkennungsschicht umfasst:
i. Spannungen werden an den nicht funktionalisierten Kanal (C₁), an das nicht funktionalisierte Tor (G₁); und an den funktionalisierten Kanal (C₂, C₃, C₄) und an das nicht funktionalisierte Tor (G₁) angelegt,
ii. dann wird ein Ausgang gemessen, wobei der Ausgang eine Differenz des Stromwerts zwischen den beiden Abflusselektroden (D₁, D₂, D₃, D4) ist.

## Revendications

1. Un capteur électrochimique organique pour la détection d'analytes dans un échantillon physiologique, le capteur comprenant :
- au moins quatre ensembles d'électrodes, chaque ensemble d'électrodes comprenant une électrode source (S₁, S₂, S₃, S₄) et une électrode drain (D₁, D₂, D₃, D₄), les électrodes source et drain de chaque ensemble étant reliées par un canal conducteur organique (C₁, C₂, C₃, C₄) et
- au moins trois grilles (G₁, G₂, G₃, G₄),
dans lequel au moins un canal (C₁) et au moins une grille (G₁) ne sont pas fonctionnalisés ;
dans lequel les canaux (C₂, C₃, C₄) et les grilles (G₂, G₃, G₄) satisfont aux trois conditions suivantes :
i. au moins un canal (C₂, C₃, C₄) ou au moins une grille (G₂, G₃, G₄) comprend une couche de reconnaissance sélective d'ions, de préférence une couche de reconnaissance sélective d'ions alcalins ;
ii. au moins un canal (C₂, C₃, C₄) ou au moins une grille (G₂, G₃, G₄) comprend une couche de reconnaissance faradique permettant la reconnaissance d'un analyte spécifique par la réduction ou l'oxydation dudit analyte au niveau dudit canal ou de ladite grille ;
iii. au moins un canal (C₂, C₃, C₄) ou au moins une grille (G₂, G₃, G₄) comprend une couche de reconnaissance d'affinité permettant la reconnaissance d'un analyte spécifique dans l'échantillon par affinité ;
dans lequel n'importe quel ensemble d'électrodes, le canal (C₁, C₂, C₃, C₄) connecté à cet ensemble d'électrodes et n'importe quelle grille (G₁, G₂, G₃, G₄) forment un transistor électrochimique organique,
**caractérisé en ce que** l'un des canaux (C₂) comprend une couche de reconnaissance sélective des ions Potassium, l'un des canaux (C₃) comprend une couche de reconnaissance sélective des ions Sodium, l'une des au moins trois grilles (G₂) comprend une couche de reconnaissance enzymatique configurée pour détecter la créatinine ; l'une des au moins trois grilles (G₃) comprend une couche de reconnaissance d'affinité configurée pour détecter le NT-pro-BNP ; et quatre des sources (Sl, S₂, S₃, S₄) sont disposées en deux paires mutualisées de sources et quatre des drains (Dl, D₂, D₃, D₄) comprennent une paire mutualisée de drains.

2. Le capteur électrochimique organique selon la revendication 1, dans lequel la couche de reconnaissance d'affinité comprend au moins l'un des éléments suivants : anticorps spécifiques, aptamères, nanocorps ou polymères à empreintes moléculaires.

3. Le capteur électrochimique organique selon l'une des revendications 1 à 2, dans lequel la couche de reconnaissance enzymatique est configurée pour effectuer une réaction en cascade multi-enzyme.

4. Le capteur électrochimique organique selon la revendication 3, comprenant en outre une grille (G₄) configurée pour la détection directe de la créatine.

5. Le capteur électrochimique organique selon la revendication 3, dans lequel la couche de reconnaissance enzymatique pour la détection de la créatinine comprend la créatininase ou la créatinine amidinohydrolase.

6. Le capteur électrochimique organique selon l'une des revendications 1 à 5, comprenant en outre une grille comportant une couche de reconnaissance enzymatique configurée pour détecter la créatine.

7. Le capteur électrochimique organique selon l'une des revendications 1 à 6, dans lequel la taille du capteur électrochimique organique est inférieure à 5 cm².

8. Dispositif électronique comprenant un capteur électrochimique organique selon l'une des revendications 1 à 7.

9. Méthode de détection d'analytes dans un échantillon physiologique, comprenant les étapes suivantes
a. Fournir le capteur électrochimique organique selon l'une des revendications 1 à 7,
b. Disposer l'échantillon sur les canaux (C₁, C₂, C₃, C₄) et les au moins trois grilles (G₁, G₂, G₃, G₄),
c. Pour chaque transistor électrochimique organique comprenant la couche de reconnaissance enzymatique ou la couche de reconnaissance d'affinité :
i. des tensions sont respectivement appliquées au canal non fonctionnalisé (C₁) et à la grille non fonctionnalisée (G₁) ; et au canal non fonctionnalisé (C₁) et à la grille fonctionnalisée (G₂, G₃, G₄),
ii. une sortie est alors mesurée, la sortie étant un changement de la valeur du courant de drain,
d. Pour chaque transistor électrochimique organique comprenant la couche de reconnaissance sélective des ions :
i. des tensions sont respectivement appliquées au canal non fonctionnalisé (C₁), à la grille non fonctionnalisée (G₁); et au canal fonctionnalisé (C₂, C₃, C₄) et à la grille non fonctionnalisée (G₁),
ii. une sortie est alors mesurée, la sortie étant une différence de valeur de courant entre les deux électrodes de drain (D₁, D₂, D₃, D₄).
